# EUROPEAN PATENT SPECIFICATION

(11) **EP 3 715 464 B1**
(45) Date of publication and mention of the grant of the patent: **05.05.2021**
(21) Application number: 19165775.8
(22) Date of filing: 28.03.2019
(51) Int. Cl.: C12P 7/08, C12P 7/02, C12P 7/40, C12M 1/107, C12M 1/00

(54) **GREENHOUSE GAS IMPROVED FERMENTATION**
TREIBHAUSGASVERBESSERTE FERMENTIERUNG
FERMENTATION AMÉLIORÉE DE GAZ À EFFET DE SERRE

(43) Date of publication of application: 30.09.2020
(73) Proprietor: DSM IP Assets B.V., 6411 TE Heerlen (NL)
(72) Inventor: NOORMAN, Hendrik Jan, 6100 AA ECHT (NL)
(74) Representative: DSM Intellectual Property

(56) References cited:
- WO-A1-2013/076294
- WO-A2-2011/139804
- US-A1- 2018 179 559

## Description

### Field

The present invention relates to a method for cultivating a microorganism capable of utilizing an organic feedstock. According to another aspect, the present invention relates to a system for cultivating a microorganism capable of utilizing an organic feedstock.

### Background

Microorganisms are used on large scale for industrial production of the microorganisms itself or metabolites produced by the microorganisms. These industrial fermentation processes and the flanking operations embedded in a factory or biorefinery, are energy intensive and CO₂ is emitted because of energy use as well as metabolism of microorganisms itself. In view of climate change there is an urgent need for industrial fermentation processes with a reduced CO₂ emission, or without CO₂ emission, or even with negative CO₂ emissions

WO2011139804 discloses to capture CO₂ from atmosphere and to convert it with hydrogen to compounds to be fed to microorganisms.

### Detailed description

According to a first aspect, the present invention relates to a method for cultivating a microorganism capable of utilizing an organic feedstock, comprising the steps of:
(i) cultivating the microorganism in one or more bioreactors (1);
(ii) capturing CO₂ from the one or more bioreactors (1), preferably in a capturing unit (2), and reducing the CO₂ to an organic feedstock, preferably in a reduction unit (3); and
(iii) feeding at least a part of the organic feedstock, preferably from the reduction unit (3) into the one or more bioreactors (1). The present inventors found that the invention provides cultivation of microorganisms with reduced emission of CO₂, or even without emission of CO₂. Furthermore, by feeding the organic feedstock into the one or more bioreactors, the carbon substrate used for growth of the microorganism, i.e. sugar, can be reduced. This means that the biomass yield per used sugar is increased, which provides an improved production process.

A 'CO₂ capturing unit' is a physical means suitable for capturing CO₂ from the from the off-gas of the one or more bioreactors (1).

A CO₂ reduction unit' is a physical means, like an apparatus or equipment, that is suitable for the reduction of CO₂ to an organic feedstock.

'Microorganisms capable of utilizing an organic feedstock as used in the present context means microorganisms that can assimilate the organic feedstock.

'Organic feedstocks' as used in the present context are carbon containing raw materials resulting from reduction of CO₂. Preferably, the present organic feedstock is derivable from CO₂. More preferably, the present organic feedstock is derivable from CO₂ using electrochemical reduction of CO₂, microbial reduction of CO₂, enzymatic process converting CO₂, or an organic synthesis process. Alternatively, organic feedstocks as used in the present context can be defined as CO₂ derived energy carrier.

In a preferred embodiment, the present organic feedstock, CO₂ derived energy carrier, is chosen from the group consisting of formic acid (HCOOH), methanol (CH₃OH), ethylene (C₂H₄), ethanol (C₂H₅OH), 1-propanol (CH₃CH₂CH₂OH) and carbon monoxide (CO). More preferably the present organic feedstock is formic acid. The advantage of using formic acid is that it can be efficiently produced from CO₂, and microorganisms could be capable of utilizing formic acid. Microorganisms can either utilize formic acid inherently, or microorganisms can be engineered to be capable of utilizing formic acid. Hence, the present microorganism is preferably capable of utilizing formic acid.

In a preferred embodiment, the present organic feedstock is not formate. The disadvantage of formate is that formate is an anion that needs to be balanced with a cation, that needs to be supplied via titration. Subsequently in the fermentation, uptake of formic acid results in the need for back-titration to balance the cation. As a result, a salt will be produced as co-product, which is highly undesired for economic and environmental reasons.

'Cultivating the microorganism' as used in the present context comprises the production of biomass and/or the production of a compound of interest, like extracellular metabolites and/or intracellular metabolites, including complex compounds such as enzymes, lipids, polysaccharides, vitamins and antibiotics.

Preferably, the fermentation is at industrial scale. More preferably, the one or more bioreactors (1) each have a volume of more than 10 liter, more preferably more than 100 liter, even more preferably more than 1000 liter, most preferably more than 10.000 liter.
Step (i) of cultivating the microorganism in one or more bioreactors (1) can be anaerobic fermentation and/or aerobic fermentation.
Step (ii) of capturing CO₂ from the one or more bioreactors (1) and reducing the CO₂ to an organic feedstock in a reduction unit (3) means capturing CO₂ from the off-gas of the one or more bioreactors (1) and reducing the captured CO₂ to organic feedstock. Capturing and reducing the CO₂ to an organic feedstock in a reduction unit (3) can be carried out by known methods and equipment. Preferably the reduction of CO₂ is electrochemical reduction of CO₂. Alternatively, reducing the CO₂ to an organic feedstock in a reduction unit (3) is photoelectrochemical reduction of CO₂, an enzymatic reduction of CO₂, or a microbial reduction of CO₂.
Step (iii) of feeding at least a part of the organic feedstock from the reduction unit into the one or more bioreactors (1) comprising feeding at least 10% (w/w) of the organic feedstock from the reduction unit (3). More preferably, feeding at least 20% (w/w), at least 30% (w/w), at least 40% (w/w), at least 50% (w/w), at least 60% (w/w), at least 70% (w/w), at least 80% (w/w), at least 90% (w/w), at least 95% (w/w) or at least 99% (w/w) of the organic feedstock from the reduction unit (3) to the bioreactors (1). More preferably 100% of the formed organic feedstock is fed into the one or more bioreactors (1). Advantageously, the present method can be optimized in that the amount of organic feedstock that is reduced from CO₂ matches the amount of organic feedstock than can be used for cofeeding into the bioreactors. This provides an improved method that reduces CO₂ emission, and can be implemented at lower costs because the needed equipment for CO₂ reduction can be smaller. On the other hand, in view of carbon pricing, it might be advantageous to reduce all CO₂ formed to organic feedstock. Any surplus of organic feedstock, i.e. feedstock than cannot be cofed to the bioreactors (1), can be used for other commercial purposes. Hence, the present method may comprise a step of collecting organic feedstock formed and packaging and/or transporting it.

In a preferred embodiment, the present method uses two or at least two bioreactors, more preferably three or at least three bioreactors, most preferably four or at least four bioreactors. The bioreactors can ferment the same microorganism, or each bioreactor can ferment a different type of microorganism. For example, in the event two bioreactors are used, the same microorganisms can be fermented, but also two different type of microorganisms can be fermented. The advantage of combining different type of microorganisms is that a combination can be chosen that efficiently makes use of waste streams from each other. Or different type organic feedstocks reduced from CO₂ can be used by the different microorganisms because one microorganism might be better in utilizing a type of organic feedstock than another. This flexibility increases the applicability of the present invention.

In a preferred embodiment, the present microorganism is chosen from the group consisting of yeast, filamentous fungi, bacteria and algae. In a further preferred embodiment, the microorganism comprises two different microorganisms from the group consisting of yeast, filamentous fungi, bacteria and algae. More preferably, the microorganism comprises three of even four different microorganisms from the group consisting of yeast, filamentous fungi, bacteria and algae. For example, the present microorganism comprises yeast and filamentous fungi. More preferably, the present method comprises cultivating yeast in one bioreactor, and cultivating a filamentous fungus in a second bioreactor.

A yeast cell is preferably a yeast belonging to the genus of *Candida, Hansenula, Kluyveromyces, Pichia, Saccharomyces, Schizosaccharomyces,* or *Yarrowia.* More preferably, the present yeast is *Kluyveromyces lactis, Saccharomyces cerevisiae, Hansenula polymorpha, Yarrowia lipolytica* or *Pichia pastoris.*

Filamentous fungi include all filamentous forms of the subdivision *Eumycota* and *Oomycota* (as defined by Hawksworth et al., In, Ainsworth and Bisby's Dictionary of The Fungi, 8th edition, 1995, CAB International, University Press, Cambridge, UK). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. Filamentous fungal strains include, but are not limited to, strains of *Acremonium, Agaricus, Aspergillus, Aureobasidium, Chrysosporium, Coprinus, Cryptococcus, Filibasidium, Fusarium, Humicola, Magnaporthe, Mucor, Myceliophthora, Neocallimastix, Neurospora, Paecilomyces, Penicillium, Piromyces, Panerochaete, Pleurotus, Schizophyllum, Talaromyces, Rasamsonia, Thermoascus, Thielavia, Tolypocladium,* and *Trichoderma.*

Preferred filamentous fungi belong to a species of an *Acremonium, Aspergillus, Chrysosporium, Myceliophthora, Penicillium, Talaromyces, Rasamsonia, Thielavia, Fusarium* or *Trichoderma* genus, and most preferably a species of *Aspergillus niger, Acremonium alabamense, Aspergillus awamori, Aspergillus foetidus, Aspergillus sojae, Aspergillus fumigatus, Talaromyces emersonii, Rasamsonia emersonii, Aspergillus oryzae, Chrysosporium lucknowense, Fusarium oxysporum, Myceliophthora thermophila, Trichoderma reesei, Thielavia terrestris* or *Penicillium chrysogenum.* A more preferred filamentous fungi belongs to the genus *Aspergillus,* more preferably the filamentous fungi belongs to the species *Aspergillus niger* or is *Aspergillus niger.*

The term "bacteria" includes both Gram-negative and Gram-positive microorganisms. Suitable bacteria may be selected from *e.g. Escherichia, Anabaena, Caulobactert, Gluconobacter, Rhodobacter, Pseudomonas, Paracoccus, Bacillus, Brevibacterium, Corynebacterium, Rhizobium (Sinorhizobium), Flavobacterium, Klebsiella, Enterobacter, Lactobacillus, Lactococcus, Methylobacterium, Staphylococcus, Streptomyces, Actinomycetes, Xanthomonas* or *Sphingomonas.* Preferably, the bacterial cell is selected from the group consisting of *B. subtilis, B. amyloliquefaciens, B. licheniformis, B. puntis, B. megaterium, B. halodurans, B. pumilus, G. oxydans, Caulobactert crescentus CB 15, Methylobacterium extorquens, Rhodobacter sphaeroides, Pseudomonas zeaxanthinifaciens, Paracoccus denitrificans, E. coli, C. glutamicum, Staphylococcus carnosus, Streptomyces lividans, Sinorhizobium melioti* and *Rhizobium radiobacter.*

The present algae are preferably chosen from the group consisting of glaucophytes, rhodoplasts and chloroplasts. More preferably the present algae are heterotrophic algae, more preferably heterotrophic algae like *Chlorella, Nannochloropsys, Nitzschia, Thraustochytrium* or *Schizochyttrium.*

In a preferred embodiment, the microorganisms according to the invention comprises at least one polynucleotide coding for a compound of interest or at least one polynucleotide coding for a compound involved in the production of a compound of interest by the cell.

The compound of interest can be any biological compound. The biological compound may be biomass or a biopolymer or metabolite. The biological compound may be encoded by a single polynucleotide or a series of polynucleotides composing a biosynthetic or metabolic pathway or may be the direct result of the product of a single polynucleotide or products of a series of polynucleotides. The biological compound may be native to the host cell or heterologous.

The term "heterologous biological compound" is defined herein as a biological compound which is not native to the cell; or a native biological compound in which structural modifications have been made to alter the native biological compound.

The term "biopolymer" is defined herein as a chain (or polymer) of identical, similar, or dissimilar subunits (monomers). The biopolymer may be any biopolymer. The biopolymer may for example be, but is not limited to, a nucleic acid, polyamine, polyol, polypeptide (or polyamide), or polysaccharide.

The biopolymer may be a polypeptide. The polypeptide may be any polypeptide having a biological activity of interest. The term "polypeptide" is not meant herein to refer to a specific length of the encoded product and, therefore, encompasses peptides, oligopeptides, and proteins. Polypeptides further include naturally occurring allelic and engineered variations of the above- mentioned polypeptides and hybrid polypeptides. The polypeptide may be native or may be heterologous to the host cell. The polypeptide may be a collagen or gelatin, or a variant or hybrid thereof. The polypeptide may be an antibody or parts thereof, an antigen, a clotting factor, an enzyme, a hormone or a hormone variant, a receptor or parts thereof, a regulatory protein, a structural protein, a reporter, or a transport protein, protein involved in secretion process, protein involved in folding process, chaperone, peptide amino acid transporter, glycosylation factor, transcription factor, synthetic peptide or oligopeptide, intracellular protein. The intracellular protein may be an enzyme such as, a protease, ceramidases, epoxide hydrolase, aminopeptidase, acylases, aldolase, hydroxylase, aminopeptidase, lipase. The polypeptide may also be an enzyme secreted extracellularly. Such enzymes may belong to the groups of oxidoreductase, transferase, hydrolase, lyase, isomerase, ligase, catalase, cellulase, chitinase, cutinase, deoxyribonuclease, dextranase, esterase. The enzyme may be a carbohydrase, e.g. cellulases such as endoglucanases, β-glucanases, cellobiohydrolases or β-glucosidases, hemicellulases or pectinolytic enzymes such as xylanases, xylosidases, mannanases, galactanases, galactosidases, pectin methyl esterases, pectin lyases, pectate lyases, endo polygalacturonases, exopolygalacturonases rhamnogalacturonases, arabanases, arabinofuranosidases, arabinoxylan hydrolases, galacturonases, lyases, or amylolytic enzymes; hydrolase, isomerase, or ligase, phosphatases such as phytases, esterases such as lipases, proteolytic enzymes, oxidoreductases such as oxidases,, transferases, or isomerases. The enzyme may be a phytase. The enzyme may be an aminopeptidase, asparaginase, amylase, a maltogenic amylase, carbohydrase, carboxypeptidase, endo-protease, metallo-protease, serine-protease catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, protein deaminase, invertase, laccase, lipase, mannosidase, mutanase, oxidase, pectinolytic enzyme, peroxidase, phospholipase, galactolipase, chlorophyllase, polyphenoloxidase, ribonuclease, transglutaminase, or glucose oxidase, hexose oxidase or monooxygenase.

Preferably the compound of interest is a heterologous product. Preferably the compound of interest is a glucose oxidase. More preferably the compound of interest is a heterologous glucose oxidase. In another preferred embodiment the compound of interest is a lipolytic enzyme, e.g. a lipolytic enzyme having one or more of the activities selected from the group consisting of: lipase (triacyl glycerol lipase), phospholipase (e.g phospholipase A1 and/or phospholipase A2 and/or phospholipase B and/or phospholipase C), galactolipase.

According to the present invention, a polypeptide or enzyme also can be a product as described in WO2010/102982. According to the present invention, a polypeptide can also be a fused or hybrid polypeptide to which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide.

Techniques for producing fusion polypeptides are known in the art, and include, ligating the coding sequences encoding the polypeptides so that they are in frame and expression of the fused polypeptide is under control of the same promoter (s) and terminator. The hybrid polypeptides may comprise a combination of partial or complete polypeptide sequences obtained from at least two different polypeptides wherein one or more may be heterologous to the host cell. Example of fusion polypeptides and signal sequence fusions are for example as described in WO2010/121933.

The biopolymer may be a polysaccharide. The polysaccharide may be any polysaccharide, including, but not limited to, a mucopolysaccharide (e. g., heparin and hyaluronic acid) and nitrogen-containing polysaccharide (eg., chitin). In a more preferred option, the polysaccharide is hyaluronic acid. In another preferred option, the polysaccharide is a hydrocolloid, e.g. xanthan, gellan, pectin, welan or another polysaccharide.

The polynucleotide coding for the compound of interest or coding for a compound involved in the production of the compound of interest according to the invention may encode an enzyme involved in the synthesis of a primary or secondary metabolite, such as organic acids, alcohols, lipids, carotenoids, beta-lactam, antibiotics, and vitamins. Such metabolite may be considered as a biological compound according to the present invention. Preferably, the present compound of interest is beta-lactam. Preferably, the present compound of interest is ethanol.

The term "metabolite" encompasses both primary and secondary metabolites; the metabolite may be any metabolite. Preferred metabolites are citric acid, gluconic acid, adipic acid, fumaric acid, itaconic acid and succinic acid.

The metabolite may be encoded by one or more genes, such as in a biosynthetic or metabolic pathway. Primary metabolites are products of primary or general metabolism of a cell, which are concerned with energy metabolism, growth, and structure. Secondary metabolites are products of secondary metabolism (see, for example, R. B. Herbert, The Biosynthesis of Secondary Metabolites, Chapman and Hall, New York, 1981).

The primary metabolite may be, but is not limited to, an amino acid, fatty acid, nucleoside, nucleotide, sugar, triglyceride, or vitamin. For example, vitamin A, B2, C, D or E.

The secondary metabolite may be, but is not limited to, an alkaloid, coumarin, flavonoid, polyketide, quinine, steroid, peptide, or terpene. The secondary metabolite may be an antibiotic, antifeedant, attractant, bacteriocide, fungicide, hormone, insecticide, or rodenticide. Preferred antibiotics are cephalosporins and beta-lactams. Other preferred metabolites are exo-metabolites. Examples of exo-metabolites are Aurasperone B, Funalenone, Kotanin, Nigragillin, Orlandin, Other naphtho-γ-pyrones, Pyranonigrin A, Tensidol B, Fumonisin B2 and Ochratoxin A.

The biological compound may also be the product of a selectable marker. A selectable marker is a product of a polynucleotide of interest which product provides for biocide or viral resistance, resistance to heavy metals, prototrophy to auxotrophs, and the like. Selectable markers include, but are not limited to, amdS (acetamidase), argB (ornithinecarbamoyltransferase), bar (phosphinothricinacetyltransferase), hygB (hygromycin phosphotransferase), niaD (nitrate reductase), pyrG (orotidine-5'-phosphate decarboxylase), sC (sulfate adenyltransferase), trpC (anthranilate synthase), ble (phleomycin resistance protein), hyg (hygromycin), NAT or NTC (Nourseothricin) as well as equivalents thereof.

According to the invention, the compound of interest is preferably a polypeptide as described in the list of compounds of interest.

Preferably, the polypeptide is an enzyme as described in the list of compounds of interest. Preferably a glucose oxidase. In another embodiment the enzyme is a lipolytic enzyme.

According to another embodiment of the invention, the compound of interest is preferably a metabolite.

According to yet another embodiment, the compound of interest is biogas.

The present microorganism may already be capable of producing the compound of interest. The microorganism may also be provided with a homologous or heterologous nucleic acid construct that encodes a polypeptide wherein the polypeptide may be the compound of interest or a polypeptide involved in the production of the compound of interest. The person skilled in the art knows how to modify a microorganism such that it is capable of producing the compound of interest.

In a further preferred embodiment, the present method further comprises the step:
(iv) electrolyzing water into H₂ and O₂ in an electrolysis unit (4), and feeding at least a part of the H₂ into the reduction unit (3) for reducing the CO₂ to the organic feedstock.
The advantage of electrolyzing water into H₂ and O₂ is that no H₂ needs to be sourced from external sources. The electricity needed for electrolysis unit (4) can advantageously be obtained from surplus electricity of other equipment on site, to provide a sustainable solution for the needed electricity. Alternatively, the electricity is generated from renewable energy, such as solar, wind or hydro energy.

In a further preferred embodiment, the present method further comprises the step:
(v) feeding at least a part of the O₂ into the one or more bioreactors (1). The O₂ is the by product of the electrolysis of water. It can advantageously be used in the one or more bioreactors, for example for aerobic fermentation. Furthermore, it was found that all aeration of the one or more bioreactors can be realized by using the O₂ from the electrolysis unit (4). Hence, no O₂ from a different source is needed anymore, which provides a further sustainability improvement. For example, no compressor for air is needed anymore, or a smaller compressor can be used, because the electrolysis unit (4) can deliver the O₂ under pressure that is comparable with the pressure by a conventional compressor. Preferably, the step (iv) of electrolyzing water into H₂ and O₂ in an electrolysis unit (4) is carried out at a pressure within the range of 0.5 to 10 bar, preferably 1 to 8 bar, more preferably 1.5 to 6 bar, most preferably 2 to 4 bar, preferably bar absolute pressure.

In a further preferred embodiment, the present method further comprises the step:
(v) feeding at least a part of the H₂ into the one or more bioreactors (1). Any surplus of H₂, i.e. H₂ that is not needed for reducing of CO₂ can advantageously be cofed into the one or more bioreactors. Microorganisms that can utilize H₂ can be fermented by cofeeding with H₂. By doing so the amount of CO₂ resulting from metabolism of the microorganism can be reduced. This provides a further sustainability advantage. Moreover, by cofeeding by H₂, the amount of sugar or other carbon containing feedstock needed for fermentation can be reduced, which provides a significant cost improvement. Hence, the present microorganism, or one of the present microorganisms, is preferably capable of utilizing H₂. More preferably, the present microorganism is capable of utilizing the present organic feedstock and H₂. Alternatively, one microorganism is capable of utilizing the present organic feedstock and another type of microorganisms is capable of utilizing H₂. For example, the present method comprises a step of fermentation a yeast capable of utilizing formic acid in one bioreactor, and fermentation a filamentous fungus capable of utilizing H₂ in another bioreactor. More specifically, the present method comprises a step of cultivating a *Saccharomyces cerevisiae* capable of utilizing formic acid in one bioreactor, and cultivating a *Penicillium chrysogenum* capable of utilizing H₂, or cultivating a *Saccharomyces cerevisiae* capable of utilizing H₂ in one bioreactor, and cultivating a *Penicillium chrysogenum* capable of utilizing formic acid in another bioreactor.

In a further preferred embodiment, cultivating a yeast produces yeast biomass, whereas cultivating a filamentous fungi produces a compound of interest. Hence, the present method preferably comprises a step of harvesting biomass or yeast biomass and/or comprises a step of harvesting a compound of interest.

The present method can be further improved by monitoring the fermentation process. In a preferred embodiment, the present method further comprises the steps of:
(vi) selecting at least one process parameter of the fermentation process, for which current measured values are determined during the course of the fermentation process;
(vii) comparing a respective current measured value of the at least one selected process parameter with a corresponding estimated value of a model parameter estimated by a process model for this at least one process parameter;
(viii) comparing a variance between the respective current measured value and the corresponding estimated value for the at least one selected process parameter with a predetermined threshold value; and
(ix) changing at least one defined model parameter employed in the process model when the predetermined threshold value is exceeded by the variance:
wherein step (vii) to (ix) with a respective changed model parameter are executed until the variance is placed below a predetermined threshold value; and optionally
wherein in cases that, after a predetermined number of repetitions of steps (vii) to (ix) the threshold value is met by the variance, the method is discontinued and a warning is generated as output.

In the one or more bioreactors (1), CO₂ capturing unit (2), CO₂ reduction unit (3), and/or in the electrolysis unit (4), various environmental and/or process parameters can be regulated and controlled for the respective fermentation process, such as pH value, temperature, CO₂ supply, H₂ supply, oxygen supply, nitrogen supply, sugar content, organic feedstock content and/or mixer settings. However, fermentation processes are biologically complex and very sensitive. Constant close monitoring of the fermentation process is therefore necessary to maintain the corresponding environmental conditions in the one or more bioreactors for a consistent and optimal course of the process and so that the biomass or the bioactive cells used in the nutrient solution grow and can produce the desired biomass and/or compound of interest.

It is favorable if a process parameter of the fermentation process, for which during the course of the fermentation process measured values can be determined approximately in real time, is selected as the process parameter to be measured. For this reason, the variance between the process model and the course of the fermentation process can be determined approximately in real time, particularly if the process model is calculated in parallel with the ongoing fermentation process. A direct comparison between the measured value of the selected process parameter and the estimated value calculated or predicted by the process model can thus be performed for the process parameter. A near-instant intervention is thus also enabled if there is an error in the fermentation process.

Expediently the repetitions of steps (vii) to (ix) of the present method are performed, i.e., comparison between measured value and estimated value of the selected process parameter. Here, comparisons of the variance with the predetermined threshold value and modification of the model parameter of the process model when the threshold value is exceeded, are performed with the respective modified model parameter at short intervals in time such as every 10 seconds. The process model can thus be rapidly adjusted, e.g., with the presence of biological variability or variability in the process control (e.g., differences in the raw materials, or fluctuations in the composition of the substrate) so that a correspondingly valid prediction of the fermentation process can be performed for the further course of the process. In case of errors in the bioprocess, a near-instant response can occur. A near-instant intervention allows damage to the bioprocess and/or to the equipment, for instance, to be avoided or prevented.

In a preferred embodiment of the invention, what is known as the respiratory quotient, also abbreviated to RQ, is selected as a process parameter that is measured during the course of the fermentation process. The respiratory quotient is a process parameter in fermentation processes, which represents an indicator of the processes within a bioactive cell. The respiratory quotient describes a ratio of the CO₂ produced at a given time to the O₂ consumed at the same time. During the course of a fermentation process, the respiratory quotient can be measured very easily in real time, e.g., using what is known as off-gas analysis.

As an alternative or additionally, a concentration of the biomass and/or a concentration of the substrate can be selected as process parameters, which are measured during the course of the fermentation process. The determination of the current measured values of the concentration of the biomass during the course of the fermentation process occurs, for instance, based on the electrical properties of the biomass. Current measured values for the concentration of the substrate (e.g., sugar etc.) can likewise be determined in the course of the fermentation process, such as based on spectroscopic properties using spectroscopy, in particular using reflection spectroscopy.

Furthermore, it is advantageous if what is known as a deterministic process model is employed to estimate the process parameters of the fermentation process. With a deterministic approach for an illustration of a fermentation process as a model, the knowledge of the respective process-specific, biochemical processes inside and outside of the biomass or cells employed is converted into mathematical equations during the course of the fermentation process.

In a further preferred embodiment, the steps (vi) to (ix) are carried out by a computer implemented method, more preferably a computer implemented method using algorithms. The aid from a computer and algorithms is beneficial in that variations in process parameters can be easily compared with data from historical fermentation processes, and that these historical data can be used for a correct interpretation of the variation in the process parameter.

Given the beneficial sustainability achievements of the present invention, the present invention relates, according to another aspect, to a system for cultivating a microorganism capable of utilizing an organic feedstock, said system comprises one or more bioreactors (1) for cultivating said microorganism, a CO₂ (2) capturing unit for capturing CO₂ from the one or more bioreactors (1), a CO₂ reduction unit (3) for reducing the CO₂ to formic acid, and/or one more conduits (10) to introduce the organic feedstock into the one more bioreactors (1).

In a preferred embodiment, the present system further comprises an electrolysis unit (4) for electrolysis of water and/or a conduit (11) to introduce H₂ from the electrolysis unit (4) to the CO₂ reduction unit (3). Preferably the present electrolysis unit (4) comprises means for pressurizing the O₂ and or the electrolysis reaction. This is advantageous in that pressurized O₂ can be introduced into the one or more bioreactors (1).

In yet another preferred embodiment, the present system further comprises one or more conduits (12) for introducing H₂ and/or O₂ into the one or more bioreactors (1). This is advantageous in that an optimal use can be made of both products, between the CO₂ reducing unit (3) and the one or more bioreactors (1). Dependent of the course of the fermentation process, the amount of H₂ feeding into the CO₂ reducing unit (3) and/or the amount of H₂ and/or O₂ feeding into the one or more bioreactors (1) can be adjusted to achieve an optimal CO₂ reduction, optimal outlet, and/or reduced intake of substrate.

In a preferred embodiment, the present system further comprises one or more inlets (13) for introducing substrate into the one or more bioreactors (1) and one or more outlets (14) for product formed in the one or more bioreactors (1).

In yet another preferred embodiment, the present system further comprises a computer implemented system for a fermentation monitoring tool for simulating and/or monitoring the fermentation method as defined in any of the preceding claims, said computer implemented system comprises at least one processor, a user interface, a control system interface configured to adjust one or more process parameters of the fermentation method, a memory comprising computer readable medium storing instructions for simulating and/or monitoring the fermentation method, wherein the instructions for simulating and/or monitoring the fermentation method configure the processor to:
(vi) selecting at least one process parameter of the fermentation method, for which current measured values are determined during the course of the fermentation method;
(vii) comparing a respective current measured value of the at least one selected process parameter of the fermentation method with a corresponding estimated value of a model parameter estimated by a process model for this at least one process parameter;
(viii) comparing a variance between the respective current measured value and the corresponding estimated value for the at least one selected process parameter with a predetermined threshold value; and
(ix) changing at least one defined model parameter employed in the process model when the predetermined threshold value is exceeded by the variance:
wherein step (vii) to (ix) with a respective changed model parameter are executed until the variance is placed below a predetermined threshold value; and optionally
wherein in cases that, after a predetermined number of repetitions of steps (vii) to (ix) the threshold value is met by the variance, the method is discontinued and a warning is generated as output on the user interface.

### Description of the figures

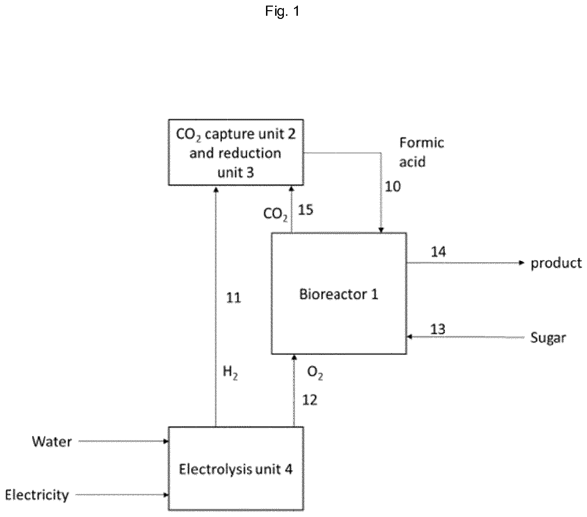

In the embodiment of figure 1, the substrate sugar is introduced via conduits 13 into bioreactor 1. The bioreactor 1 provides the conditions allowing fermentation of microorganisms capable of utilizing organic feedstock. The produced product, like the microorganism or a compound of interests produced by the microorganisms, leaves the bioreactors 1 via conduits 14. CO₂ formed in the bioreactors 1 is introduced into a CO₂ capture unit 2 via conduits 15. In reduction unit 3 the CO₂ is reduced towards organic feedstock, utilizing H₂. The organic feedstock is introduced into bioreactors 1 via conduits 10, enabling fermentation of the microorganisms that are capable to utilize the organic feedstock. The advantage is that CO₂ is not released into the environment, and the amount of sugar needed for the fermentation process can be reduced. The H₂ needed for reduction of CO₂ into organic feedstock can be introduced into reduction 3 and can be sourced from a supplier. Preferably, the electrolysis unit 4 electrolyzes water into H₂ and O₂. The H₂ can be introduced into reduction unit 3 via conduit 11. The O₂ can be introduced into bioreactor 1. Alternatively, air can be introduced into bioreactor 1 for aerobic processes.

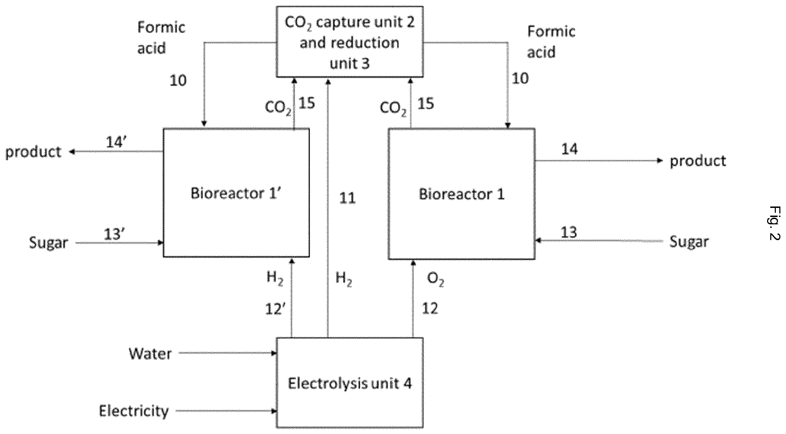

In the embodiment of figure 2, in addition to the embodiment of figure 1, a second bioreactor 1' is present. The electrolysis unit 4 electrolyzes water into H₂ and O₂. The H₂ can be introduced into reduction unit 3 via conduit 11. Advantageously, H₂ can be introduced in a bioreactor 1' for fermentation of microorganisms capable of utilizing H₂. This is advantageous in that H₂ cofeeding reduces the amount of sugar needed, and can reduce the amount of CO₂ formed by the microorganisms in bioreactor 1'.

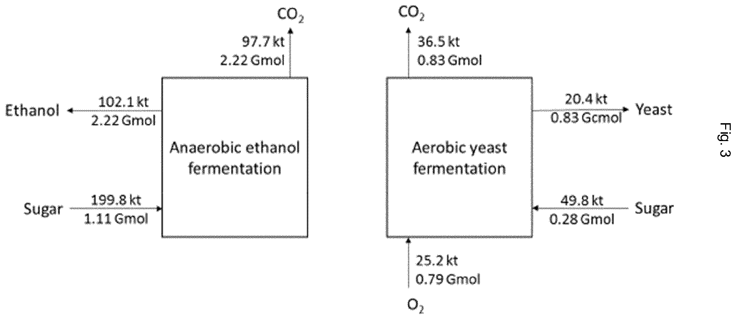

Figure 3 shows a calculation of required substrate and O₂, the produced ethanol and yeast, and the emitted CO₂, in a conventional system.

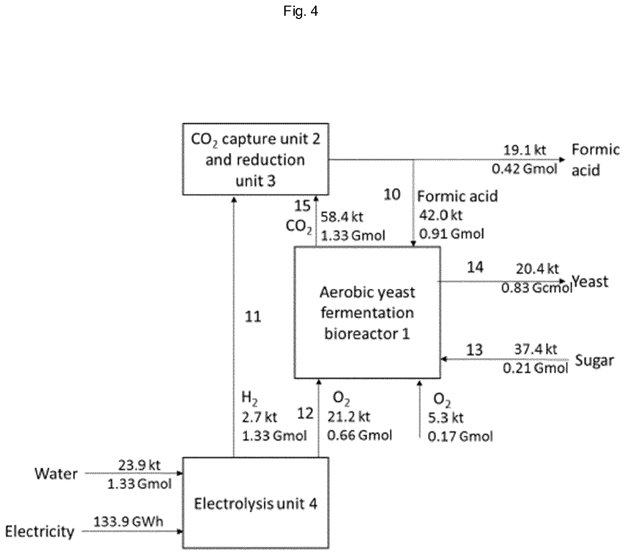

In the embodiment of figure 4, aerobic yeast bioreactor 1 produces yeast via outlet 14, using sugar via inlet 13 and O₂ via inlet 12. The O₂ is produced by electrolysis unit 4. The electrolysis unit 4 produces H₂ that is introduced in reduction unit 3 via conduit 11. CO₂ from the aerobic yeast bioreactor 1 is captured in unit 2, via conduit 15. The CO₂ is reduced to formic acid in reduction unit 3 and introduced into yeast bioreactor 1 via conduit 10.

In operation, the embodiment of figure 4 allows production of the same amount of yeast as in figure 3. However, no CO₂ is released into the environment, and the amount of sugar and O₂ needed for yeast fermentation is significantly reduced.

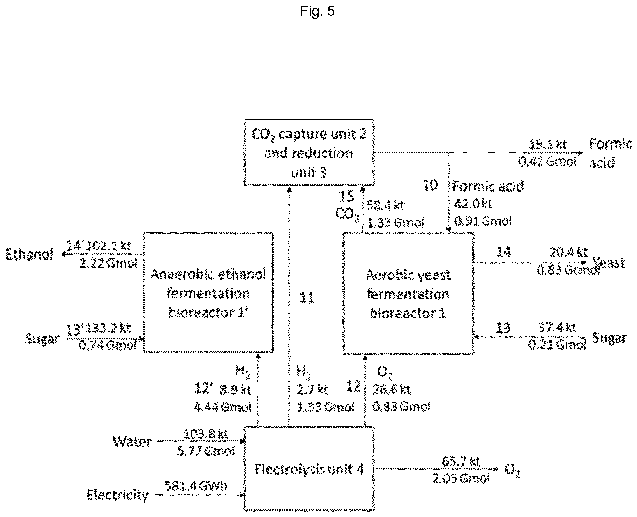

In the embodiment of figure 5, an aerobic yeast fermentation in bioreactor 1 is combined with anaerobic ethanol fermentation in bioreactor 1'. O₂ produced by electrolysis unit 4 is introduced into aerobic yeast bioreactor 1 via conduit 12. H₂ produced by electrolysis unit 4 is introduced into anaerobic ethanol bioreactor 1' via conduit 12' and into reduction unit 3 via conduit 11. CO₂ is only captured from aerobic yeast bioreactor 1 via conduit 15. Formic acid is introduced in aerobic yeast bioreactor 1 only via conduit 10, and any surplus formic acid is collected for external use.

In operation, the embodiment of figure 5 allows the production of ethanol without production of CO₂. Further the amount of sugar needed for production of ethanol is reduced due to feeding the H₂. The aerobic yeast fermentation produces the same amount of yeast using a reduced amount of sugar. No CO₂ is released into the environment. The O₂ needed for fermentation is totally derived from the electrolysis unit. Hence, figure 5 shows a system allowing a CO₂ neutral fermentation process.

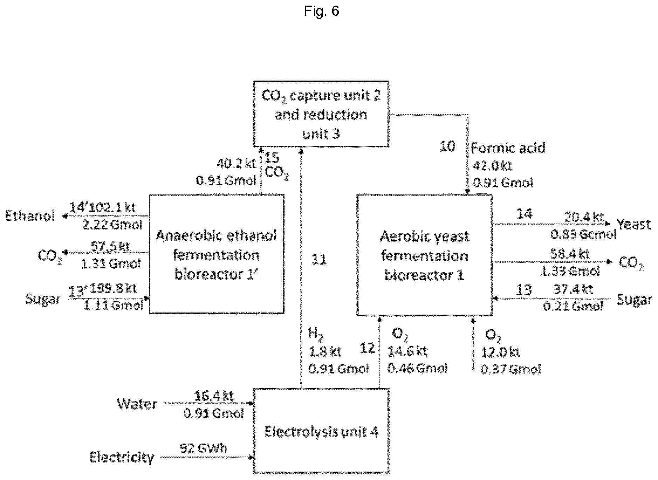

In the embodiment of figure 6, an aerobic yeast fermentation in bioreactor 1 is combined with anaerobic ethanol fermentation in bioreactor 1'. O₂ produced by electrolysis unit 4 is introduced into aerobic yeast bioreactor 1 via conduit 12. H₂ produced by electrolysis unit 4 is introduced into reduction unit 3 via conduit 11. CO₂ is only captured from anaerobic ethanol bioreactor 1' via conduit 15. Formic acid is introduced in aerobic yeast bioreactor 1 only via conduit 10.

In operation, the embodiment of figure 6 only produces the amount of formic acid required for the aerobic yeast fermentation in bioreactor 1. Any surplus CO₂ from both bioreactors 1 that is not needed for production of formic acid is released. This embodiment allows a reduction of sugar needed for fermentation of yeast, while it minimizes the energy needed for electrolysis of water in electrolysis unit 4.

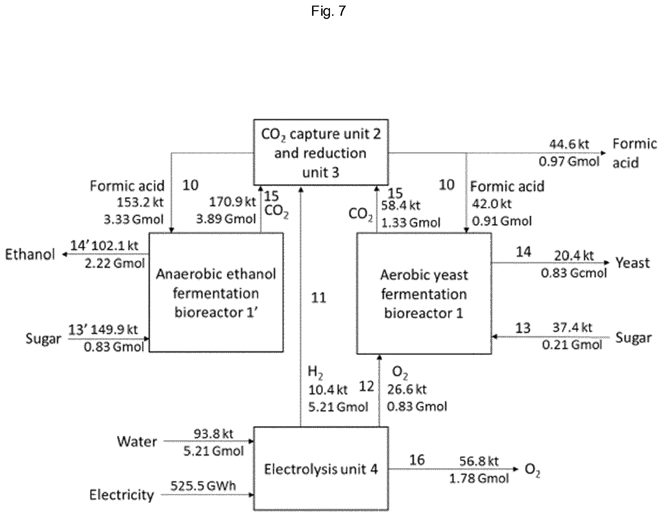

In the embodiment of figure 7, aerobic yeast bioreactor 1 produces yeast via outlet 14, using sugar via inlet 13 and O₂ via inlet 12. Anaerobic ethanol fermentation bioreactor 1' produces ethanol via outlet 14' using sugar via inlet 13'. CO₂ from both the aerobic yeast bioreactor 1 and anaerobic ethanol fermentation bioreactor 1' is captured in unit 2, via conduits 15. The CO₂ is reduced to formic acid in reduction unit 3 and introduced into both the aerobic yeast bioreactor 1 and anaerobic ethanol fermentation bioreactor 1' using conduits 10. Any surplus formic acid is collected via an outlet on conduit 10. Electrolysis unit 4 electrolyzes water into H₂ and O₂. The H₂ is introduced in reduction unit 3 via conduit 11. The O₂ is introduced in the aerobic yeast fermentation bioreactor 1. Any surplus O₂ leaves the system via conduit 16.

In operation, the embodiment of figure 7 reaches ethanol and yeast production using minimal amounts of sugar. All CO₂ is captured and reduced to formic acid. The microorganisms grown in the bioreactors are capable in utilizing formic acid.

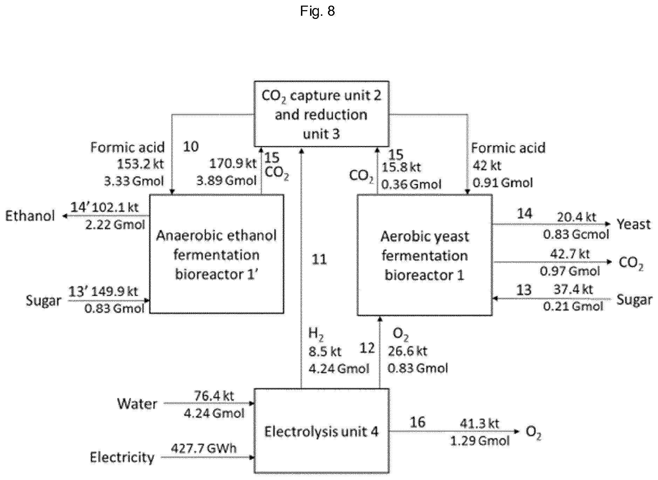

In the embodiment of figure 8, CO₂ from both the aerobic yeast bioreactor 1 and anaerobic ethanol fermentation bioreactor 1' is captured in unit 2, via conduits 15. The CO₂ is reduced to formic acid in reduction unit 3 and introduced into both the aerobic yeast bioreactor 1 and anaerobic ethanol fermentation bioreactor 1' using conduits 10. Electrolysis unit 4 electrolyzes water into H₂ and O₂. The H₂ is introduced in reduction unit 3 via conduit 11. The O₂ is introduced in the aerobic yeast fermentation bioreactor 1. Any surplus O₂ leaves the system via conduit 16.

In operation, the embodiment of figure 8 reaches ethanol and yeast production using minimal amounts of sugar. The amount of CO₂ that is captured and reduced to formic acid is adapted to the amount needed for the production of formic acid. Any CO₂ surplus is emitted. The microorganisms grown in the bioreactors only need to be capable in utilizing formic acid. In this embodiment, the electricity is reduced to what is needed for the minimal electrolysis of water for formation of formic acid.

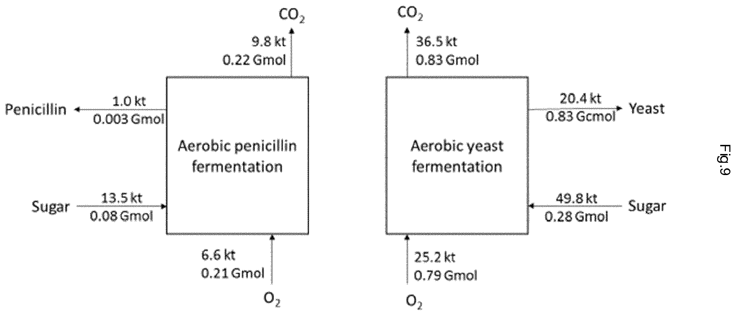

Figure 9 shows a calculation of required substrate and O₂, the produced penicillin and yeast, and the emitted CO₂, in a conventional system.

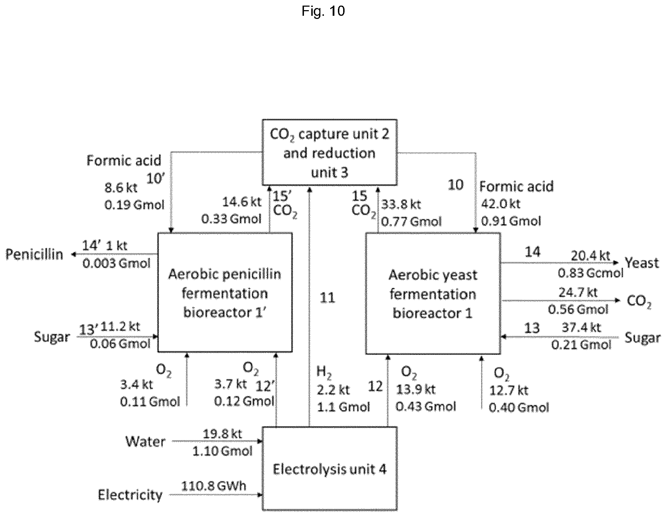

Figure 10 shows an embodiment of the invention wherein CO₂ from both the aerobic yeast bioreactor 1 and aerobic penicillin bioreactor 1' is captured in unit 2 via conduits 15. The CO₂ is reduced to formic acid in reduction unit 3 and introduced into both the aerobic yeast bioreactor 1 and aerobic penicillin bioreactor 1' using conduits 10. Electrolysis unit 4 electrolyzes water into H₂ and O₂. The H₂ is introduced in reduction unit 3 via conduit 11. The O₂ is introduced in the aerobic yeast fermentation bioreactor 1 and the aerobic penicillin bioreactor 1'. The yeast in bioreactor 1 is capable of utilizing formic acid. The penicillium in bioreactor 1' is capable of utilizing formic acid.

In operation, the embodiment of figure 10 produces the same amount of penicillium and yeast as in comparative figure 9, while the amount of sugar needed to produce the same yield is reduced from 63.3 kt to 48.6 kt, and the amount of externally supplied O₂ is reduced from 31.8 kt to 16.1 kt.

## Claims

1. Method for cultivating a microorganism capable of utilizing an organic feedstock, comprising the steps of:
(i) cultivating the microorganism in one or more bioreactors (1);
(ii) capturing CO₂ from the one or more bioreactors (1) and reducing the CO₂ to an organic feedstock in a reduction unit (3); and
(iii) feeding at least a part of the organic feedstock from the reduction unit (3) into the one or more bioreactors (1).

2. Method according to claim 1, wherein the organic feedstock is chosen from the group consisting of formic acid, methanol, ethylene, ethanol, 1-propanol and carbon monoxide.

3. Method according to claim 1 or claim 2, wherein the organic feedstock is formic acid.

4. Method according to any of the preceding claims, wherein the microorganism is chosen from the group consisting of yeast, filamentous fungi, bacteria and heterotrophic algae.

5. Method according to any of the preceding claims, further comprising the step:
(iv) electrolyzing water into H₂ and O₂ in an electrolysis unit (4), and feeding at least a part of the H₂ into the reduction unit (3) for reducing the CO₂ to the organic feedstock.

6. Method according to claim 5, further comprising the step:
(v) feeding at least a part of the O₂ into the one or more bioreactors (1).

7. Method according to claim 5 or claim 6, further comprising the step:
(v) feeding at least a part of the H₂ into the one or more bioreactors (1).

8. Method according to any of the preceding claims, wherein the microorganism capable of utilizing an organic feedstock comprises at least one polynucleotide coding for a compound of interest or at least one polynucleotide coding for a compound involved in the production of a compound of interest by the cell.

9. Method according to any of the preceding claims, comprising monitoring the fermentation process comprising the steps of:
(vi) selecting at least one process parameter of the fermentation process, for which current measured values are determined during the course of the fermentation process;
(vii) comparing a respective current measured value of the at least one selected process parameter with a corresponding estimated value of a model parameter estimated by a process model for this at least one process parameter;
(viii) comparing a variance between the respective current measured value and the corresponding estimated value for the at least one selected process parameter with a predetermined threshold value; and
(ix) changing at least one defined model parameter employed in the process model when the predetermined threshold value is exceeded by the variance:
wherein step (vii) to (ix) with a respective changed model parameter are executed until the variance is placed below a predetermined threshold value; and optionally
wherein in cases that, after a predetermined number of repetitions of steps (vii) to (ix) the threshold value is met by the variance, the method is discontinued and a warning is generated as output.

10. System for cultivating a microorganism capable of utilizing an organic feedstock, said system comprises one or more bioreactors (1) for cultivating said microorganism, a CO₂ (2) capturing unit for capturing CO₂ from the one or more bioreactors (1), a CO₂ reduction unit (3) for reducing the CO₂ to organic feedstock, and one more conduits (10) to introduce the organic feedstock into the one more bioreactors (1).

11. System according to the preceding claim, further comprising an electrolysis unit (4) for electrolysis of water and a conduit (11) to introduce H₂ from the electrolysis unit (4) to the CO₂ reduction unit (3).

12. System according to any of the preceding claims, further comprising one or more conduits (12) for introducing H₂ and/or O₂ into the one or more bioreactors (1).

13. System according to any of the preceding claims, further comprising one or more inlets (13) for introducing substrate into the one or more bioreactors (1) and one or more outlets (14) for product formed in the one or more bioreactors (1).

14. System according to any of the preceding claims, further comprising a computer implemented system for a fermentation simulation tool for simulating the fermentation method as defined in any of the preceding claims, said computer implemented system comprises at least one processor, a user interface, a control system interface configured to adjust one or more process parameters of the fermentation method, a memory comprising computer readable medium storing instructions for simulating the fermentation method, wherein the instructions for simulating the fermentation method configure the processor to:
(vi) selecting at least one process parameter of the fermentation method, for which current measured values are determined during the course of the fermentation method;
(vii) comparing a respective current measured value of the at least one selected process parameter of the fermentation method with a corresponding estimated value of a model parameter estimated by a process model for this at least one process parameter;
(viii) comparing a variance between the respective current measured value and the corresponding estimated value for the at least one selected process parameter with a predetermined threshold value; and
(ix) changing at least one defined model parameter employed in the process model when the predetermined threshold value is exceeded by the variance:
wherein step (vii) to (ix) with a respective changed model parameter are executed until the variance is placed below a predetermined threshold value; and optionally
wherein in cases that, after a predetermined number of repetitions of steps (vii) to (ix) the threshold value is met by the variance, the method is discontinued and a warning is generated as output on the user interface.

## Patentansprüche

1. Verfahren zum Kultivieren eines Mikroorganismus, der zur Verwendung eines organischen Einsatzmaterials fähig ist, umfassend die folgenden Schritte:
(i) das Kultivieren des Mikroorganismus in einem oder mehreren Bioreaktoren (1);
(ii) das Auffangen von CO₂ aus dem einen oder den mehreren Bioreaktoren (1) und das Reduzieren des CO₂ zu einem organischen Einsatzmaterial in einer Reduktionseinheit (3) und
(iii) das Zuführen mindestens eines Teils des organischen Einsatzmaterials aus der Reduktionseinheit (3) in den einen oder die mehreren Bioreaktoren (1).

2. Verfahren nach Anspruch 1, wobei das organische Einsatzmaterial aus der aus Ameisensäure, Methanol, Ethylen, Ethanol, 1-Propanol und Kohlenmonoxid bestehenden Gruppe ausgewählt ist.

3. Verfahren nach Anspruch 1 oder Anspruch 2, wobei es sich beim organischen Einsatzmaterial um Ameisensäure handelt.

4. Verfahren nach einem der vorhergehenden Ansprüche, wobei der Mikroorganismus aus der aus Hefe, Fadenpilzen, Bakterien und heterotrophen Algen bestehenden Gruppe ausgewählt ist.

5. Verfahren nach einem der vorherigen Ansprüche, das weiterhin den folgenden Schritt umfasst:
(iv) das Elektrolysieren von Wasser zu H₂ und O₂ in einer Elektrolyseeinheit (4) und das Zuführen mindestens eines Teils des H₂ in die Reduktionseinheit (3), um das CO₂ zum organischen Einsatzmaterial zu reduzieren.

6. Verfahren nach Anspruch 5, das weiterhin den folgenden Schritt umfasst:
(v) das Zuführen mindestens eines Teils des O₂ in den einen oder die mehreren Bioreaktoren (1).

7. Verfahren nach Anspruch 5 oder Anspruch 6, das weiterhin den folgenden Schritt umfasst:
(v) das Zuführen mindestens eines Teils des H₂ in den einen oder die mehreren Bioreaktoren (1).

8. Verfahren nach einem der vorhergehenden Ansprüche, wobei der zur Verwendung eines organischen Einsatzmaterials fähige Mikroorganismus mindestens ein Polynucleotid, das für eine interessierende Verbindung codiert, oder mindestens ein Polynucleotid umfasst, das für eine Verbindung codiert, das an der Erzeugung einer interessierenden Verbindung durch die Zelle beteiligt ist.

9. Verfahren nach einem der vorhergehenden Ansprüche, welches das Überwachen des Fermentationsverfahrens umfasst, umfassend die folgenden Schritte:
(vi) das Auswählen mindestens eines Verfahrensparameters des Fermentationsverfahrens, bei dem aktuelle Messwerte während des Fermentationsverfahrens bestimmt werden;
(vii) das Vergleichen eines jeweiligen aktuellen Messwerts des mindestens einen ausgewählten Verfahrensparameters mit einem entsprechenden Schätzwert eines Modellparameters, der durch ein Verfahrensmodell für diesen mindestens einen Verfahrensparameter geschätzt wird;
(viii) das Vergleichen einer Varianz zwischen dem jeweiligen aktuellen Messwert und dem entsprechenden Schätzwert für den mindestens einen ausgewählten Verfahrensparameter mit einem vorbestimmten Schwellenwert und
(ix) das Ändern mindestens eines im Verfahrensmodell verwendeten definierten Modellparameters, wenn der vorbestimmte Schwellenwert von der Varianz überschritten wird:
wobei Schritt (vii) bis (ix) mit einem jeweils geänderten Modellparameter ausgeführt werden, bis die Varianz einen vorbestimmten Schwellenwert unterschreitet; und
wobei gegebenenfalls in Fällen, in denen nach einer vorbestimmten Zahl von Wiederholungen der Schritte (vii) bis (ix) der Schwellenwert von der Varianz erreicht wird, das Verfahren beendet und eine Warnung als Ausgabe erzeugt wird.

10. System zum Kultivieren eines zur Verwendung eines organischen Einsatzmaterials fähigen Mikroorganismus, wobei das System einen oder mehrere Bioreaktoren (1) zum Kultivieren des Mikroorganismus, eine CO₂-Auffangeinheit (2) zum Auffangen von CO₂ aus dem einen oder den mehreren Bioreaktoren (1), eine CO₂-Reduktionseinheit (3) zum Reduzieren des CO₂ zum organischen Einsatzmaterial und ein oder mehrere Leitungen (10) zum Einführen des organischen Einsatzmaterials in den einen oder die mehreren Bioreaktoren (1) umfasst.

11. System nach dem vorhergehenden Anspruch, das weiterhin eine Elektrolyseeinheit (4) zur Elektrolyse von Wasser und eine Leitung (11) zum Einführen von H₂ aus der Elektrolyseeinheit (4) in die CO₂-Reduktionseinheit (3) umfasst.

12. System nach einem der vorhergehenden Ansprüche, das weiterhin eine oder mehrere Leitungen (12) zum Einführen von H₂ und/oder O₂ in den einen oder die mehreren Bioreaktoren (1) umfasst.

13. System nach einem der vorhergehenden Ansprüche, das weiterhin einen oder mehrere Einlässe (13) zum Einführen von Substrat in den einen oder die mehreren Bioreaktoren (1) und einen oder mehrere Auslässe (14) für in dem einen oder den mehreren Bioreaktoren (1) gebildetes Produkt umfasst.

14. System nach dem vorhergehenden Anspruch, das weiterhin ein computerimplementiertes System für ein Fermentationssimulationstool zum Simulieren des Fermentationsverfahrens gemäß der Definition in einem der vorhergehenden Ansprüche umfasst, wobei das computerimplementierte System mindestens einen Prozessor, eine Benutzerschnittstelle, eine Steuersystemschnittstelle, die so konfiguriert ist, dass sie einen oder mehrere Verfahrensparameter des Fermentationsverfahrens einstellt, einen ein computerlesbares Medium umfassenden Speicher umfasst, der Anweisungen zum Simulieren des Fermentationsverfahrens umfasst, wobei die Anweisungen zum Simulieren des Fermentationsverfahrens den Prozessor so konfigurieren, dass:
(vi) mindestens ein Verfahrensparameter des Fermentationsverfahrens ausgewählt wird, bei dem aktuelle Messwerte während des Fermentationsverfahrens bestimmt werden;
(vii) ein jeweiliger aktueller Messwert des mindestens einen ausgewählten Verfahrensparameters des Fermentationsverfahrens mit einem entsprechenden Schätzwert eines Modellparameters verglichen wird, der durch ein Verfahrensmodell für diesen mindestens einen Verfahrensparameter geschätzt wird;
(viii) das Vergleichen einer Varianz zwischen dem jeweiligen aktuellen Messwert und dem entsprechenden Schätzwert für den mindestens einen ausgewählten Verfahrensparameter mit einem vorbestimmten Schwellenwert und
(ix) das Ändern mindestens eines im Verfahrensmodell verwendeten definierten Modellparameters, wenn der vorbestimmte Schwellenwert von der Varianz überschritten wird:
wobei Schritt (vii) bis (ix) mit einem jeweils geänderten Modellparameter ausgeführt werden, bis die Varianz einen vorbestimmten Schwellenwert unterschreitet; und
wobei gegebenenfalls in Fällen, in denen nach einer vorbestimmten Zahl von Wiederholungen der Schritte (vii) bis (ix) der Schwellenwert von der Varianz erreicht wird, das Verfahren beendet und eine Warnung als Ausgabe auf der Benutzerschnittstelle erzeugt wird.

## Revendications

1. Procédé de culture d'un micro-organisme capable d'utiliser une matière première organique, comprenant les étapes de :
(i) culture du micro-organisme dans un ou plusieurs bioréacteurs (1) ;
(ii) capture de CO₂ provenant d'un ou de plusieurs bioréacteurs (1) et réduction du CO₂ en une matière première organique dans une unité de réduction (3) ; et
(iii) introduction d'au moins une partie de la matière première organique de l'unité de réduction (3) dans le ou les bioréacteurs (1).

2. Procédé selon la revendication 1, la matière première organique étant choisie dans le groupe constitué par l'acide formique, le méthanol, l'éthylène, l'éthanol, le 1-propanol et le monoxyde de carbone.

3. Procédé selon la revendication 1 ou la revendication 2, la matière première organique étant l'acide formique.

4. Procédé selon l'une quelconque des revendications précédentes, le micro-organisme étant choisi dans le groupe constitué par la levure, des champignons filamenteux, des bactéries et des algues hétérotrophes.

5. Procédé selon l'une quelconque des revendications précédentes, comprenant en outre l'étape :
(iv) électrolyse de l'eau en H₂ et O₂ dans une unité d'électrolyse (4), et introduction d'au moins une partie du H₂ dans l'unité de réduction (3) pour réduire le CO₂ en matière première organique.

6. Procédé selon la revendication 5, comprenant en outre l'étape :
(v) introduction d'au moins une partie de l'O₂ dans le ou les bioréacteurs (1).

7. Procédé selon la revendication 5 ou la revendication 6, comprenant en outre l'étape :
(v) introduction d'au moins une partie de H₂ dans le ou les bioréacteurs (1).

8. Procédé selon l'une quelconque des revendications précédentes, le micro-organisme capable d'utiliser une matière première organique comprenant au moins un polynucléotide codant pour un composé d'intérêt ou au moins un polynucléotide codant pour un composé impliqué dans la production d'un composé d'intérêt par la cellule.

9. Procédé selon l'une quelconque des revendications précédentes, comprenant le contrôle du processus de fermentation comprenant les étapes de :
(vi) sélection d'au moins un paramètre de processus du processus de fermentation, pour lequel des valeurs mesurées actuelles sont déterminées au cours du processus de fermentation ;
(vii) comparaison d'une valeur mesurée actuelle respective de l'au moins un paramètre de processus sélectionné avec une valeur estimée correspondante d'un paramètre de modèle estimé par un modèle de processus pour cet au moins un paramètre de processus ;
(viii) comparaison d'une variance entre la valeur mesurée actuelle respective et la valeur estimée correspondante pour l'au moins un paramètre de processus sélectionné avec une valeur seuil prédéterminée ; et
(ix) modification d'au moins un paramètre de modèle défini utilisé dans le modèle de processus lorsque la valeur seuil prédéterminée est dépassée par la variance :
les étapes (vii) à (ix) avec un paramètre de modèle modifié respectif étant exécutées jusqu'à ce que la variance se retrouve en dessous d'une valeur seuil prédéterminée ; et éventuellement
dans les cas où, après un nombre prédéterminé de répétitions des étapes (vii) à (ix), la valeur seuil est atteinte par la variance, le procédé étant interrompu et un avertissement étant généré en sortie.

10. Système pour cultiver un micro-organisme capable d'utiliser une matière première organique, ledit système comprenant un ou plusieurs bioréacteurs (1) pour cultiver ledit micro-organisme, une unité de capture de CO₂ (2) pour capturer le CO₂ provenant du ou des bioréacteurs (1), une unité de réduction de CO₂ (3) pour réduire le CO₂ en matière première organique, et un ou plusieurs conduits (10) pour introduire la matière première organique dans le ou les bioréacteurs (1).

11. Système selon la revendication précédente, comprenant en outre une unité d'électrolyse (4) pour l'électrolyse de l'eau et un conduit (11) pour introduire l'H₂ de l'unité d'électrolyse (4) dans l'unité de réduction de CO₂ (3).

12. Système selon l'une quelconque des revendications précédentes, comprenant en outre un ou plusieurs conduits (12) pour introduire l'H₂ et/ou l'O₂ dans le ou les bioréacteurs (1).

13. Système selon l'une quelconque des revendications précédentes, comprenant en outre une ou plusieurs entrées (13) pour introduire un substrat dans le ou les bioréacteurs (1), et une ou plusieurs sorties (14) pour le produit formé dans le ou les bioréacteurs (1).

14. Système selon l'une quelconque des revendications précédentes, comprenant en outre un système mis en œuvre par ordinateur pour un outil de simulation de fermentation pour simuler le procédé de fermentation selon l'une quelconque des revendications précédentes, ledit système mis en œuvre par ordinateur comprenant au moins un processeur, une interface utilisateur, une interface de système de commande configurée pour ajuster un ou plusieurs paramètres de processus du procédé de fermentation, une mémoire comprenant un support lisible par ordinateur stockant des instructions pour simuler le procédé de fermentation, les instructions pour simuler le procédé de fermentation configurant le processeur pour :
(vi) sélectionner au moins un paramètre de processus du procédé de fermentation, pour lequel des valeurs mesurées actuelles sont déterminées au cours du procédé de fermentation ;
(vii) comparer une valeur mesurée actuelle respective de l'au moins un paramètre de processus sélectionné du procédé de fermentation avec une valeur estimée correspondante d'un paramètre de modèle estimé par un modèle de processus pour cet au moins un paramètre de processus ;
(viii) comparer une variance entre la valeur mesurée actuelle respective et la valeur estimée correspondante pour l'au moins un paramètre de processus sélectionné avec une valeur seuil prédéterminée ; et
(ix) modifier au moins un paramètre de modèle défini utilisé dans le modèle de processus lorsque la valeur seuil prédéterminée est dépassée par la variance :
les étapes (vii) à (ix) avec un paramètre de modèle modifié respectif étant exécutées jusqu'à ce que la variance se retrouve en dessous d'une valeur seuil prédéterminée ; et éventuellement
dans les cas où, après un nombre prédéterminé de répétitions des étapes (vii) à (ix), la valeur seuil est atteinte par la variance, le procédé étant interrompu et un avertissement étant généré en tant que sortie sur l'interface utilisateur.
